# EUROPEAN PATENT SPECIFICATION

(11) **EP 2 227 153 B1**
(45) Date of publication and mention of the grant of the patent: **11.05.2016**
(21) Application number: 08868291.9
(22) Date of filing: 18.12.2008
(51) Int. Cl.: A61B 17/22, A61M 25/10

(54) **TWO-PART EXTRACTION BALLOON**
ZWEITEILIGER EXTRAKTIONSBALLON
BALLONNET D'EXTRACTION EN DEUX PARTIES

(30) Priority: 27.12.2007 US 16976 P
(43) Date of publication of application: 15.09.2010
(73) Proprietor: Cook Medical Technologies LLC, Bloomington, IN 47404 (US)
(72) Inventor: GAYZIK, Caroline, M., Winston-Salem North Carolina 27104 (US)
(74) Representative: Garratt, Peter Douglas
(86) International application number: PCT/US2008/087456
(87) International publication number: WO 2009/085971

(56) References cited:
- EP-A- 2 002 779
- DE-A1- 3 438 131
- US-A- 4 295 464
- US-A- 4 627 837
- US-A- 4 696 668
- US-A- 5 417 657
- US-A- 6 056 721
- US-B1- 6 692 484

## Description

### TECHNICAL FIELD

The present invention relates to medical devices, and in particular balloon catheters useful in performing extraction procedures within a mammalian patient.

### BACKGROUND OF THE INVENTION

Balloon catheters are used in a variety of medical procedures. For example, certain types of balloon catheters are used to dilate strictures or deploy stents within a duct of the patient. These types of balloon catheters typically utilize a non-compliant balloon material that is configured to be inflated to a predetermined diameter. Inflation fluid is delivered to the interior of the balloon under a relatively high pressure, thereby allowing the balloon to compress the stricture or deploy the stent.

Other types of balloon catheters utilize a compliant balloon material. Compliant balloon catheters are often used to extract material from the duct of a patient. For example, compliant balloons are often used to sweep stones or other debris out of the common bile duct and into the duodenum, where it can pass out of the body naturally. Compliant balloons are relatively flexible to allow the balloon to conform to the size and shape of the duct in which the extraction procedure is being performed.

Some compliant balloons are capable of being inflated to a range of diameters. This reduces the number of balloon catheters that may be required to perform a particular extraction procedure. Nevertheless, certain extraction procedures may require the use of multiple balloon catheters of varying sizes, or size ranges. For example, a relatively large diameter balloon catheter may be required to sweep a stone along the common bile duct towards the papilla of vater. This balloon, however, may be too large to pass through the papilla while still inflated, or even partially inflated. But a deflated balloon is incapable of sweeping the stone through the papilla. Thus, the first balloon must be removed and replaced with a smaller diameter balloon to finish the procedure by sweeping the stone through the papilla and into the duodenum.

Exchanging balloon catheters during the medical procedure has a number of drawbacks. For example, removing and replacing a balloon catheter with a different size balloon catheter is a time consuming process that increases the duration and complicates the medical procedure. In addition, the stone or debris being extracted may get pushed back up the duct by the second balloon catheter as it is advanced up into the duct during the exchange.

What is needed is a compliant balloon catheter that is capable of extracting stones or other debris though a duct or bodily lumen of varying shapes and diameters.

Reference is directed to US 4 627 837 in which there is provided a catheter device equipped at its distal portion with a pair of balloons inflatable from the proximal end of the catheter. One balloon completely covers the distal tip of the catheter to serve, when inflated, as an anchor to retain the catheter in an appropriate ampulla and as a cushion to prevent internal tissue damage. The second balloon serves, when inflated, to remove and dislodge impacted biliary stones. Reference is further directed to US 6 056 721 in which there is provided a balloon catheter device for treating an obstructing material within a vascular conduit or other body passageway. A high compliance balloon and a spaced apart angioplasty balloon are coaxially disposed along the distal end. Reference is also directed to US 5 417 657 in which there is provided a no-sepsis urinary catheter, comprising three lumens, each in fluid communication with a drainage tip for receiving urine from the bladder, a retention balloon for retaining the catheter within the bladder, and a microporous bacteriostasis balloon for the diffusion by osmosis of a pharmaceutical agent for the killing and prevention of bacteria growth within and around the bladder to preclude the development of sepsis therein. Reference is also directed to US 6 692 484 in which there is provided a device for extracting biliary or urinary stones, calculi or the like (stones) from the biliary or urinary tract of a patient including a catheter shaft, a first expandable apparatus, such as a balloon, for dilating the tract entrance or the sphincter thereat, and a second expandable apparatus, which may also be a balloon, for capturing the stone and/or urging it out of the tract. Reference is further directed to DE 34 38 131 in which there is provided a device for extraction of stones with a balloon or balloons at the front part of the catheter, which balloon or balloons can be dilated by fluid thereof, the rounded parts of the balloons being connected/connectable with threads for reinforcement in the longitudinal direction. Reference is also directed to US 4 295 464 in which there is provided a ureteric stone extractor wherein an inner dislodger catheter is slidable within a relatively larger outer dialator catheter. A dislodger balloon is eccentrically attached to the inner catheter and can be inflated once the inner catheter is positioned above and beyond the arrested stone. The outer dilator catheter has a cylindrical dilator balloon attached thereto to effect distension and dilation of the ureter below the arrested stone, thereby providing a sufficient spacial area into which the stone can be moved. Reference is further directed to US 4 696 668 in which there is provided a double balloon multiple lumen nasobiliary occlusion catheter including two inflatable balloons positioned about the distal end of the catheter. When inflated, the balloons occlude the lumen of the bile duct at two points thereby creating a sealed space between the balloons into which a treatment solution may be infused. Since this space is sealed from the remaining biliary tree, the treatment solution will find access to the gallbladder and any stones therein via the cystic duct with the exclusion of bile from the gallbladder fundus.

### SUMMARY OF THE INVENTION

Accordingly, the present invention comprises a balloon catheter as defined in claim 1.

According to one aspect of the invention, the balloon catheter comprises a first balloon and a second balloon attached to an elongate catheter shaft near the distal end thereof. The first balloon has an inflated diameter that is larger than the inflated diameter of the second balloon, and in particular has an inflated working diameter range that is greater than the inflated working diameter range of the second balloon. Each of the balloons comprises an elastic or compliant material, and is separately inflatable via one of two inflation lumens extending through the catheter shaft. The inflation lumens are each configured to be attached to a source of inflation fluid. In one exemplary embodiment, the larger first balloon is located adjacent to and proximal of the smaller second balloon. The larger first balloon is configured to engage and sweep calculi or debris through a relatively large bodily lumen, duct or passageway, whereas the smaller second balloon is configured to engage and sweep calculi or debris through a relatively small bodily lumen, duct or passageway. The shaft of the balloon catheter may include additional lumens for the injection of contrast or the passage of a wire guide therethrough.

According to an example, a method of extracting calculi or debris from a bodily lumen or duct is provided. The method includes the step of providing a balloon catheter having a pair of compliant balloons, wherein one balloon has an inflated working diameter that is larger than the inflated diameter of the other balloon. The balloon catheter is introduced into a first portion of the bodily duct until the pair of balloons is positioned "upstream" of the calculi or debris. The larger balloon is inflated so as to engage the interior surface of the duct. The balloon catheter is then withdrawn in a proximal direction until the larger balloon engages and sweeps the calculi or debris along the first portion of the duct. The smaller balloon is then inflated and the larger balloon is deflated. The balloon catheter is then further withdrawn in a proximal direction until the smaller balloon engages and sweeps the calculi or debris along a second portion of the bodily duct, the second portion of the duct having a smaller internal diameter than that of the first portion of the duct. A wire guide may be employed to guide the balloon catheter during its initial introduction into the bodily duct. Contrast may be injected through the balloon catheter and into the bodily duct to allow fluoroscopic viewing of the calculi or debris.

The above-described two-part balloon catheter permits a single balloon catheter to extract calculi or debris from a bodily duct having a relatively large range of internal diameters and/or shapes, and thereby eliminates the need for multiple balloon catheters of different sizes and/or shapes.

These and other advantages, as well as the invention itself, will become apparent in the details of construction and operation as more fully described below. Moreover, it should be appreciated that several aspects of the invention can be used with other types of wire guides, wire guided catheters, and other medical devices.

### BRIEF DESCRIPTION OF THE DRAWINGS

Embodiments of the present invention will now be described by way of example with reference to the accompanying drawings, in which:
Figure 1 illustrates a perspective view of an exemplary embodiment of the two-part extraction balloon of the present invention;
Figure 2 is an illustration showing the two-part extraction balloon of Figure 1 configured to sweep calculi along the common bile duct; and
Figure 3 is an illustration showing the two-part extraction balloon of Figure 1 configured to sweep calculi though the sphincter of oddi.

### DESCRIPTION OF THE INVENTION

The invention is described with reference to the drawings in which like elements are referred to by like numerals. The relationship and functioning of the various elements of this invention are better understood by the following detailed description. However, the embodiments of this invention as described below are by way of example only, and the invention is not limited to the embodiments illustrated in the drawings. It should also be understood that the drawings are not to scale and in certain instances details have been omitted, which are not necessary for an understanding of the present invention, such as conventional details of fabrication and assembly.

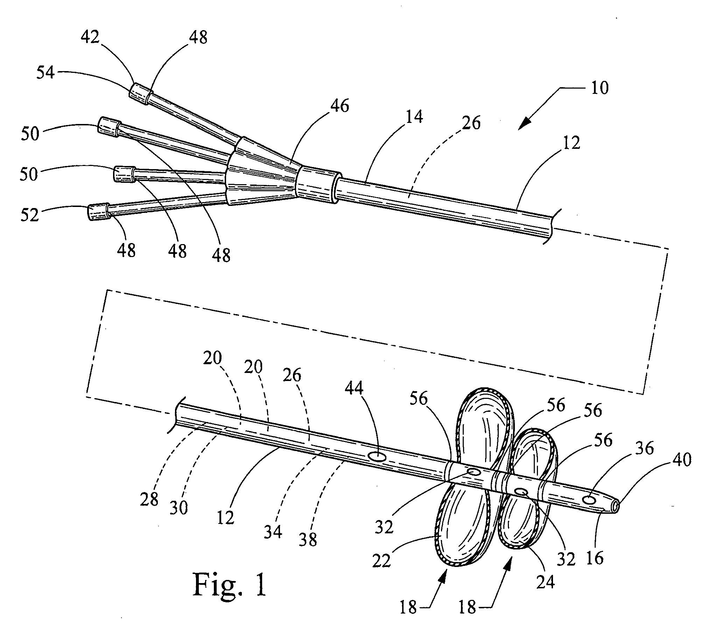

In general, Figure 1 illustrates an exemplary embodiment of the two-part extraction balloon 10 of the present invention. The two-part extraction balloon 10 comprises an elongate catheter shaft 12 extending between a proximal end 14 and a distal end 16. A plurality of compliant balloons 18 is affixed to the catheter shaft 12 near the distal end 16 thereof. Each of the balloons 18 is in fluid communication with an inflation lumen 20 extending through the catheter shaft 12. As will be explained in greater detail below, each of the balloons 18 may be independently inflated by introducing an inflation fluid through an inflation lumen 20 associated with the respective balloon 18.

In the particular embodiment illustrated, the two-part extraction balloon 10 comprises a first balloon 22 and a second balloon 24, both of which comprise an elastic or compliant material such as natural rubber latex. The first balloon 22 is located proximally of the second balloon 24, and has an inflated diameter or range of diameters that is greater than that of the second balloon 24. In particular, the first balloon 22 has inflated working diameter that ranges between 15-20 mm, and more specifically has three discrete inflated diameters of 15, 18 and 20 mm. The second balloon 24 has an inflated working diameter that ranges between 8.5-15 mm, and more specifically has three discrete inflated diameters of 8.5, 12 and 15 mm.

The specific inflated diameter of the first or second balloon 22, 24 is achieved by introducing a predetermined amount of inflation fluid into the interior volume of the first or second balloon 22, 24. In general, the upper and lower limits (i.e., working range) of the first and second balloon inflated diameter is dependent on the size, shape and type of balloon material utilized. Inflating the first or second balloon 22, 24 to a diameter larger than the upper end of the working range may cause the balloon to rupture. In addition, an over inflated balloon may be too rigid to conform to the shape of the patient's duct, thereby inhibiting passage of the balloon therethrough. Inflating the first or second balloon 22, 24 to a diameter smaller than the lower end of the working range, also referred to as the nominal diameter, will generally result in a balloon profile that is incapable of performing the intended function. More specifically, an under inflated balloon will lack the appropriate shape and/or rigidity to enable the sweeping of calculi along the duct of a mammalian patient. In addition, it may be difficult to determine the exact diameter of an under inflated balloon. In other words, it is certainly possible to only partially inflate the larger, first balloon 22 so as to achieve a diameter that falls within the range of the smaller, second balloon 24, for example 12 mm. However, the under inflated first balloon 22 will not have the desired shape or rigidity to enable the first balloon 22 to sweep calculi along the duct of a patient.

As set forth above, the first and second balloons 22, 24 provide a range of working diameters that is larger than would be possible if only a single balloon were provided. As will be explained in greater detail below in connection with a description of an exemplary procedure utilizing the two-part extraction balloon 10 of the present invention, the greater range of working diameters provided by the plurality of balloons 18 allows the apparatus to be used to sweep calculi or other debris through ducts or structures of varying diameters without needing to utilize and/or exchange multiple balloon catheters of different sizes.

Although the embodiment illustrated in Fig. 1 comprises a first and second balloon 22, 24, it should be appreciated that more than two balloons could be utilized. The use of three or more balloons would further increase the overall working diameter range of the apparatus. Likewise, although the first and second balloons 22, 24 each comprise a generally circular inflated diameter, other shapes could be utilized. For example, the second balloon 24 could comprise an oval or elliptical shape to match correspondingly shaped ducts within the mammalian patient. The arrangement of the balloons 18 could also be altered so that the inflated diameter of the second balloon 24 is greater than that of the first balloon 22.

The elongate catheter shaft 12 comprises a flexible plastic material that has been extruded to form the desired shape. In the particular embodiment illustrated, which is intended for biliary applications, the shaft 12 has an overall length of 200 cm and a diameter of 7 French along a substantial portion thereof. The distal portion of the shaft tapers down to 5 French to facilitate introduction through the working channel of an endoscope and into the patient. The distal end 16 of the shaft 12 may also be rounded or tapered to provide an atraumatic tip. The shaft 12 may be transparent or translucent to allow observation of the other devices extending therethrough, such as wire guides. Other materials and dimensions may be utilized for the shaft 12 depending on the application or procedure for which the apparatus is intended. For example, the shaft 12 may comprise separate proximal and distal shaft sections manufactured from different materials, such as a metal proximal shaft section and plastic distal shaft section. Two-part catheter shafts of this type are often utilized for vascular applications requiring stiff proximal shaft sections and flexible distal shaft sections.

The catheter shaft 12 comprises one or more radiopaque markers 56 disposed near the distal end 16 thereof. In the particular embodiment illustrated, the catheter shaft 12 comprises three radiopaque markers 56 disposed adjacent to each end of the first and second balloons 22, 24. As will be explained in greater detail below, these markers 56 are used to fluoroscopically determine the position of the first and second balloons 22, 24 within the patient's bodily lumen or duct. Suitable markers 56 may comprise gold or other high-density materials that are viewable with a fluoroscope.

The catheter shaft 12 comprises a plurality of lumens 26 extending therethrough. At a minimum, the shaft 12 comprises one inflation lumen 20 for each balloon 18. Thus, the embodiment of Fig. 1 includes a first inflation lumen 28 that is in fluid communication with the first balloon 22, and a second inflation lumen 30 that is in fluid communication with the second balloon 24. The first and second lumens 28, 30 each comprise a port or opening 32 through the side wall of the shaft 12 in communication with the interior of the first and second balloons 22, 24, respectively. Inflation fluid passes through the lumen 28, 30 and into or out of the balloon 22, 24 via the opening 32 so as to inflate or deflate the balloon 22, 24.

In the particular embodiment illustrated, the shaft 12 further comprises a third lumen 34 for the injection of contrast material. The third lumen 34 terminates in an opening or port 36 near the distal end 16 of the shaft 12, and distally of the first and second balloons 22, 24. This allows contrast material to be injected into the patient's duct distally of the balloons 22, 24, which may be inflated to direct or contain the contrast within the duct "upstream" from the balloons 22, 24. This arrangement permits the "upstream" portion of the patient's duct to be observed using fluoroscopy. Alternatively, the contrast port 36 may be located proximally of the first and second balloons 22, 24. Such an arrangement would direct or contain the contrast within the duct "downstream" from the balloons 22, 24, which allows the "downstream" portion of the duct to be observed under fluoroscopy. Two separate contrast lumens may also be provided, wherein one lumen terminates in a port distal of the balloons and the other lumen terminates in a port proximal of the balloons.

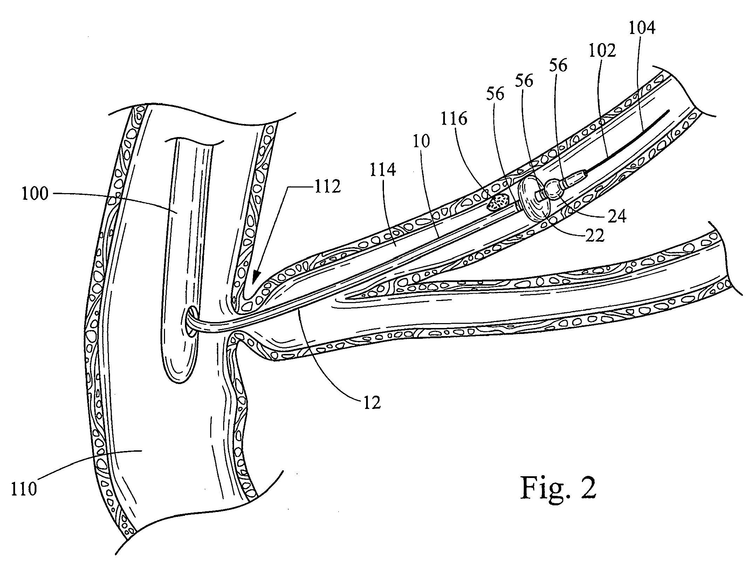

In the particular embodiment illustrated, the shaft 12 further comprises a wire guide lumen 38. The wire guide lumen 38 is configured to allow a wire guide 102 to slidably extend therethrough (see Figs. 2-3). As is well understood by those skilled in the art, wire guides are relatively flexible, elongate devices that are often used to gain initial access into a patient's duct or target region within the patient. Once the distal end of the wire guide has been positioned within the target region, for example within the common bile duct, the wire guide can subsequently be used to guide additional devices into the target region. This is typically accomplished by advancing the additional device over the wire guide. For example, the proximal end of the wire guide may be advanced into and through the wire guide lumen 38 of the shaft 12. The two-part extraction balloon 10 is then pushed along the wire guide, which is held stationary, until the distal end 16 of the shaft 12 reaches the target region within the patient. The wire guide lumen 38 may also be configured to receive a stylet or stiffening member disposed therethrough. Stylets and stiffening members are often employed to enhance the stiffness and/or "pushability" of the shaft 12.

In the particular embodiment illustrated, the wire guide lumen 38 comprises a distal port 40 in the distal end 16 of the shaft 12, and a proximal port 42 in the proximal end 14 of the shaft 12. An intermediate port 44 is also provided a short distance proximally of the balloons 22, 24. The intermediate port 44 allows the wire guide 102 to be coupled only to the distal-most portion of the shaft 12, which allows for the use of a shorter wire guide. Additional ports may also be utilized at other locations. In addition, the shaft 12 may comprise a splittable material adjacent to the wire guide lumen 38 that is configured to allow a wire guide to be laterally removed from the wire guide lumen 38. Alternatively, the shaft 12 may comprise a wall that has open channel, a slot or a weakened portion (by, for example, perforations or a groove).

A hub 46 is connected to the proximal end 14 of the shaft 12. The hub 46 includes a plurality of connectors 48 that are in fluid communication with the lumens 26 of the catheter shaft 12. The connectors 48 are configured for attachment to other medical devices, the introduction of other medical devices and/or the introduction or withdrawal of fluids or medications. For example, the two connectors 50 that are in fluid communication with the inflation lumens 28, 30 are configured to be connected to a source on inflation fluid, such as syringe. In particular, these connectors 50 each include a female luer fitting that is adapted to be connected to a male luer fitting on the syringe. Likewise, the connector 52 that is in fluid communication with the contrast lumen 34 may comprise a female luer fitting adapted for connection to a medical syringe or other device for supplying contrast or other fluids. The connector 54 that is in fluid communication with wire guide lumen 38 may also comprise a female luer fitting adapted to be connected to a stylet or other medical device.

The connectors 48 are preferably color coded or marked with distinguishing indicia (e.g., numerals or labels) so as to enable the user to identify which connectors 48 are associated with specific lumens 26. The connectors 48 may also have different lengths and/or shapes to improve identification and handling thereof. Moreover, it should be appreciated that the locations of one or more of the connectors 48 can be distal to the hub 46. For example, the connector 54 in fluid communication with the wire guide lumen 38 could be located along the shaft 12 and spaced a short distance distally of the hub 46. Such a location may provide improved handling of a wire guide 102 disposed therethrough.

An exemplary method of using the two-part extraction balloon 10 of the present invention will now be described with reference to Figs. 2-3, which illustrate the extraction (sweeping) of calculi from the common bile duct of a mammalian patient. As shown in Fig. 2, an endoscope 100 is inserted into the patient and advanced until the distal end is positioned in the duodenum 110 and adjacent to the papilla of vater 112. A wire guide 102 is advanced through the working (accessory) channel of the endoscope 100 until the distal end 104 thereof extends outwardly from the distal end of the endoscope 100. The wire guide 102 is then further advanced through the papilla 112 and into the common bile duct 114 until the distal end 104 of the wire guide 102 is disposed in within the target region of the patient, e.g., near calculi or other debris. The wire guide 102 may then be used to advance the two-part extraction balloon 10 through the endoscope 100 and into the common bile duct 114. The distal end 16 of the two-part extraction balloon 10 is then positioned so that the first and second balloons 22, 24 are each disposed distally or "upstream" from the calculi 116. This may be accomplished by fluoroscopically observing the position of the radiopaque markers 56 disposed near the distal end of the catheter shaft 12. The first balloon 22 is then inflated to a diameter sufficient to substantially engage the interior wall of the duct 114, as shown in Fig. 2. The two-part extraction balloon 10 is then withdrawn in a proximal direction until the first balloon 22 engages the calculi 116. Further proximal movement of the two-part extraction balloon 10 moves or "sweeps" the calculi 116 towards the papilla 112.

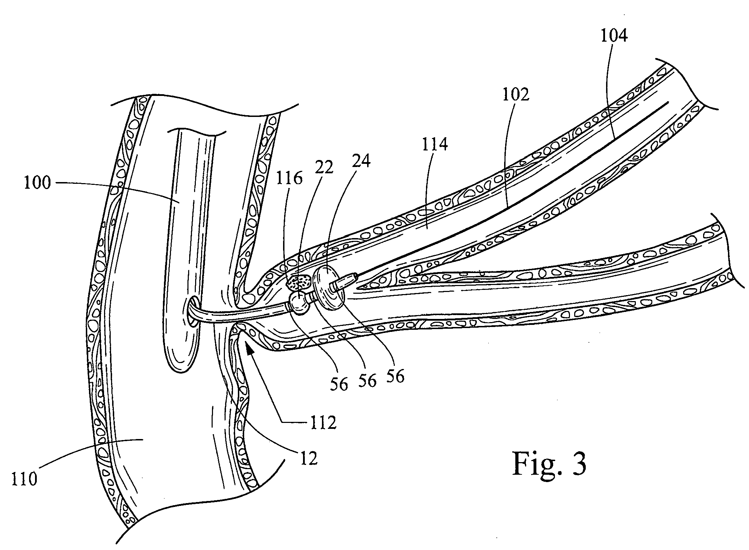

As shown in Fig. 3, the two-part extraction balloon 10 is withdrawn until the calculi 116 have been moved to a location adjacent to the papilla 112. This may be accomplished by fluoroscopically observing the position of the radiopaque markers 56 on either side of the balloons 22, 24. The second balloon 24 is then inflated and the first balloon 22 is subsequently deflated. The two-part extraction balloon 10 is then withdrawn in a proximal direction until the second 24 balloon engages the calculi 116. Further proximal movement of the two-part extraction balloon 10 moves or "sweeps" the calculi 116 through the papilla 112 and into the duodenum 110, where it can pass out of the body naturally or be removed with another medical device, such as a forceps or extraction basket.

In the exemplary procedure described above, the two-part extraction balloon 10 may be used to inject contrast into the common bile duct 114 to enable identification of calculi or other debris requiring extraction. This is typically done prior to engaging and sweeping the calculi from the common bile duct. However, the two-part extraction balloon 10 may be used to inject contrast into the common bile duct 114 at other stages of the procedure or for other purposes. For example, contrast may be injected into the common bile duct 114 to observe any strictures that may be present.

The exemplary procedure described above may also utilize other medical devices in addition to the two-part extraction balloon 10 of the present invention. For example, a sphincterotome or needle knife may be used to cut the sphincter of oddi so as to gain access through the papilla of vater 112. An ERCP catheter may be used to inject contrast media into the common bile duct 114. A lithotripter or extraction basket may be used to break up or remove calculi. The use of these other devices is well known to those skilled in the art.

Moreover, it should be appreciated that the two-part extraction balloon 10 may be employed during other medical procedures, or within other bodily lumens or ducts within the patient. For example, the above-described procedure could be reversed by first using the smaller balloon to sweep an object through a relatively small duct, and then by using the larger balloon to sweep the object through a relatively large duct. The present invention is therefore not limited to the exemplary procedure described above.

## Claims

1. A two-part extraction balloon comprising: an elongate catheter shaft extending between a proximal end and a distal end; a first expandable balloon affixed to the catheter shaft near the distal end, the first expandable balloon comprising a compliant material and expandable to a first maximum working diameter; and a second expandable balloon affixed to the catheter shaft near the distal end, the second expandable balloon comprising a compliant material and expandable to a second maximum working diameter, wherein the first maximum working diameter is greater than the second maximum working diameter,
wherein the first expandable balloon is disposed adjacent to the second expandable balloon,
wherein the first expandable balloon and the second expandable balloon are each configured to engage calculi within a patient's internal bodily lumen, and
wherein the first expandable balloon and the second expandable balloon are each configured to sweep calculi along the patient's internal bodily lumen upon proximal movement of the catheter shaft.

2. The two-part extraction balloon according to claim 1, wherein the first expandable balloon is disposed proximal of the second expandable balloon.

3. The two-part extraction balloon according to claim 1, wherein the catheter shaft comprises a first inflation lumen in fluid communication with the first expandable balloon, and further comprises a second inflation lumen in fluid communication with the second expandable balloon.

4. The two-part extraction balloon according to claim 3, wherein a hub is operably connected to the proximal end of the catheter shaft, the hub comprising a plurality of connectors in fluid communication with the first and second inflation lumens, the plurality of connectors each configured for attachment to a source of inflation fluid.

5. The two-part extraction balloon according to claim 3 further comprising a wire guide lumen configured to slidably receive a wire guide therethrough.

6. The two-part extraction balloon according to claim 5, wherein the wire guide lumen extends between a distal wire guide port in the distal end of the catheter shaft and a proximal wire guide port near the proximal end of the catheter shaft.

7. The two-part extraction balloon according to claim 6, wherein the catheter shaft comprises an intermediate wire guide port in fluid communication with the wire guide lumen, the intermediate wire guide port being located proximal of the first and second expandable balloons and a substantial distance distal of the proximal wire guide port.

8. The two-part extraction balloon according to claim 3 further comprising a contrast lumen configured for the passage of contrast therethrough, the contrast lumen terminating in a contrast port extending through a side wall of the catheter shaft and disposed near the first and second expandable balloons.

9. The two-part extraction balloon according to claim 1, wherein the first expandable balloon has a first inflated cross-sectional shape and the second expandable balloon has a second inflated cross-sectional shape, the first inflated cross- sectional shape being different than the second inflated cross-sectional shape.

10. The two-part extraction balloon according to claim 1, wherein the first expandable balloon is configured to engage a first bodily lumen having a first interior cross-section, and the second expandable balloon is configured to engage a second bodily lumen having a second interior cross-section, the second interior cross-section being smaller than the first interior cross-section.

11. The two-part extraction balloon according to claim 1, wherein the first expandable balloon comprises a first inflated working diameter range and the second expandable balloon has a second inflated working diameter range, the first inflated working diameter range being greater than the second inflated working diameter range.

12. The two-part extraction balloon according to claim 11, wherein the first inflated working diameter range is approximately 15-20 mm, and the second inflated working diameter range is approximately 8.5-15 mm.

13. The two-part extraction balloon according to claim 2, wherein the two-part extraction balloon is configured to extract calculi from a patient's common bile duct, wherein the catheter shaft has a length sufficient to extend through the papilla of vater and into the patient's common bile duct, wherein the first expandable balloon is configured to engage an interior surface of the patient's common bile duct when inflated, and wherein the second expandable balloon is configured to pass through the papilla of vater when inflated to sweep calculi through the sphincter of oddi and into the duodenum.

## Patentansprüche

1. Zweiteiliger Extraktionsballon, umfassend:
einen langgestreckten Katheterschaft, der sich zwischen einem proximalen Ende und einem distalen Ende erstreckt; einen ersten expandierbaren Ballon, der an dem Katheterschaft neben dem distalen Ende befestigt ist, wobei der erste expandierbare Ballon ein nachgiebiges Material umfasst und bis zu einem ersten maximalen Arbeitsdurchmesser expandierbar ist; und einen zweiten expandierbaren Ballon, der an dem Katheterschaft neben dem distalen Ende befestigt ist, wobei der zweite expandierbare Ballon ein nachgiebiges Material umfasst und bis zu einem zweiten maximalen Arbeitsdurchmesser expandierbar ist, wobei der erste maximale Arbeitsdurchmesser größer als der zweite maximale Arbeitsdurchmesser ist,
wobei der erste expandierbare Ballon neben dem zweiten expandierbaren Ballon angeordnet ist,
wobei der erste expandierbare Ballon und der zweite expandierbare Ballon jeweils ausgelegt sind, in Calculi in einem inneren Körperlumen eines Patienten einzugreifen, und
wobei der erste expandierbare Ballon und der zweite expandierbare Ballon jeweils ausgelegt sind, bei proximaler Bewegung des Katheterschafts Calculi entlang des inneren Körperlumens des Patienten zu streifen.

2. Zweiteiliger Extraktionsballon nach Anspruch 1, wobei der erste expandierbare Ballon proximal von dem zweiten expandierbaren Ballon angeordnet ist.

3. Zweiteiliger Extraktionsballon nach Anspruch 1, wobei der Katheterschaft ein erstes Aufblaslumen in Fluidverbindung mit dem ersten expandierbaren Ballon umfasst, und ferner ein zweites Aufblaslumen in Fluidverbindung mit dem zweiten expandierbaren Ballon umfasst.

4. Zweiteiliger Extraktionsballon nach Anspruch 3, wobei ein Nabenansatz mit dem proximalen Ende des Katheterschafts in Wirkverbindung steht, wobei der Nabenansatz eine Vielzahl von Verbindungsgliedern in Fluidverbindung mit den ersten und zweiten Aufblaslumen umfasst, wobei die Vielzahl von Verbindungsgliedern jeweils zur Befestigung an einer Aufblasflüssigkeitsquelle ausgelegt ist.

5. Zweiteiliger Extraktionsballon nach Anspruch 3, ferner umfassend ein Führungsdrahtlumen, das zur gleitenden Aufnahme eines Führungsdrahts durch es hindurch ausgelegt ist.

6. Zweiteiliger Extraktionsballon nach Anspruch 5, wobei sich das Führungsdrahtlumen zwischen einer distalen Führungsdrahtöffnung im distalen Ende des Katheterschafts und einer proximalen Führungsdrahtöffnung neben dem proximalen Ende des Katheterschafts erstreckt.

7. Zweiteiliger Extraktionsballon nach Anspruch 6, wobei der Katheterschaft eine Führungsdrahtzwischenöffnung in Fluidverbindung mit dem Führungsdrahtlumen umfasst, wobei die Führungsdrahtzwischenöffnung proximal von den ersten und zweiten expandierbaren Ballons und in einem erheblichen Abstand distal von der proximalen Führungsdrahtöffnung angeordnet ist.

8. Zweiteiliger Extraktionsballon nach Anspruch 3, ferner umfassend ein Kontrastmittellumen, das zur Durchleitung eines Kontrastmittels durch es hindurch ausgelegt ist, wobei das Kontrastmittellumen in einer Kontrastmittelöffnung endet, die sich durch eine Seitenwand des Katheterschafts erstreckt und neben den ersten und zweiten expandierbaren Ballons angeordnet ist.

9. Zweiteiliger Extraktionsballon nach Anspruch 1, wobei der erste expandierbare Ballon eine erste aufgeblasene Querschnittsgestalt aufweist und der zweite expandierbare Ballon eine zweite aufgeblasene Querschnittsgestalt aufweist, wobei sich die erste aufgeblasene Querschnittsgestalt von der zweiten aufgeblasenen Querschnittsgestalt unterscheidet.

10. Zweiteiliger Extraktionsballon nach Anspruch 1, wobei der erste expandierbare Ballon ausgelegt ist, in ein erstes Körperlumen mit einem ersten Innenquerschnitt einzugreifen, und der zweite expandierbare Ballon ausgelegt ist, in ein zweites Körperlumen mit einem zweiten Innenquerschnitt einzugreifen, wobei der zweite Innenquerschnitt kleiner als der erste Innenquerschnitt ist.

11. Zweiteiliger Extraktionsballon nach Anspruch 1, wobei der erste expandierbare Ballon einen ersten aufgeblasenen Arbeitsdurchmesserbereich umfasst und der zweite expandierbare Ballon einen zweiten aufgeblasenen Arbeitsdurchmesserbereich umfasst, wobei der erste aufgeblasene Arbeitsdurchmesserbereich größer als der zweite aufgeblasene Arbeitsdurchmesserbereich ist.

12. Zweiteiliger Extraktionsballon nach Anspruch 11, wobei der erste aufgeblasene Arbeitsdurchmesserbereich ungefähr 15-20 mm beträgt, und der zweite aufgeblasene Arbeitsdurchmesserbereich ungefähr 8,5-15 mm beträgt.

13. Zweiteiliger Extraktionsballon nach Anspruch 2, wobei der zweiteilige Extraktionsballon ausgelegt ist, Calculi aus dem Gallengang zu extrahieren, wobei der Katheterschaft eine Länge aufweist, die ausreicht, um sich durch die Vater-Papille und in den Gallengang des Patienten zu erstrecken, wobei der erste expandierbare Ballon ausgelegt ist, in eine Innenfläche des Gallengangs des Patienten nach dem Aufblasen einzugreifen, und wobei der zweite expandierbare Ballon ausgelegt ist, nach dem Aufblasen durch die Vater-Papille zu verlaufen, um Calculi durch den Oddi-Sphinkter und in das Duodenum zu streifen.

## Revendications

1. Ballonnet d'extraction en deux parties, comprenant : une gaine de cathéter allongée s'étendant entre une extrémité proximale et une extrémité distale ; un premier ballonnet dilatable fixé à la gaine de cathéter près de l'extrémité distale, le premier ballonnet dilatable comprenant une matière souple et pouvant se dilater jusqu'à un premier diamètre maximal de fonctionnement ; et un second ballonnet dilatable fixé à la gaine de cathéter près de l'extrémité distale, le second ballonnet dilatable comprenant une matière souple et pouvant se dilater jusqu'à un second diamètre maximal de fonctionnement, dans lequel premier diamètre maximal de fonctionnement est supérieur au second diamètre maximal de fonctionnement,
dans lequel le premier ballonnet dilatable est disposé adjacent au second ballonnet dilatable,
dans lequel le premier ballonnet dilatable et le second ballonnet dilatable sont chacun configurés pour venir au contact de calculs à l'intérieur d'une lumière corporelle interne d'un patient, et
dans lequel le premier ballonnet dilatable et le second ballonnet dilatable sont chacun configurés pour balayer les calculs le long de la lumière corporelle interne du patient lors du mouvement proximal de la gaine de cathéter.

2. Ballonnet d'extraction en deux parties selon la revendication 1, dans lequel le premier ballonnet dilatable est disposé en position proximale par rapport au second ballonnet dilatable.

3. Ballonnet d'extraction en deux parties selon la revendication 1, dans lequel la gaine de cathéter comprend une première lumière de gonflage en communication fluidique avec le premier ballonnet dilatable, et comprend en outre une seconde lumière de gonflage en communication fluidique avec le second ballonnet dilatable.

4. Ballonnet d'extraction en deux parties selon la revendication 3, dans lequel un raccord est relié fonctionnellement à l'extrémité proximale de la gaine de cathéter, le raccord comprenant une pluralité de connecteurs en communication fluidique avec les première et seconde lumières de gonflage, la pluralité de connecteurs étant chacun configurés pour se fixer à une source de fluide de gonflage.

5. Ballonnet d'extraction en deux parties selon la revendication 3, comprenant en outre une lumière de passe-fil configurée pour recevoir à coulissement à travers elle un passe-fil.

6. Ballonnet d'extraction en deux parties selon la revendication 5, dans lequel la lumière de passe-fil s'étend entre un orifice distal de passe-fil dans l'extrémité distale de la gaine de cathéter et un orifice proximal de passe-fil près de l'extrémité proximale de la gaine de cathéter.

7. Ballonnet d'extraction en deux parties selon la revendication 6, dans lequel la gaine de cathéter comprend un orifice intermédiaire de passe-fil en communication fluidique avec la lumière de passe-fil, l'orifice intermédiaire de passe-fil étant situé en position proximale par rapport aux premier et second ballonnets dilatables et à une distance considérable en position distale par rapport à l'orifice proximal de passe-fil.

8. Ballonnet d'extraction en deux parties selon la revendication 3, comprenant en outre une lumière de contraste configurée pour faire passer par elle un produit de contraste, la lumière de contraste se terminant par un orifice de contraste traversant une paroi latérale de la gaine de cathéter et disposé près des premier et second ballonnets dilatables.

9. Ballonnet d'extraction en deux parties selon la revendication 1, dans lequel le premier ballonnet dilatable présente une première forme gonflée en coupe transversale et le second ballonnet dilatable présente une seconde forme gonflée en coupe transversale, la première forme gonflée en coupe transversale étant différente de la seconde forme gonflée en coupe transversale.

10. Ballonnet d'extraction en deux parties selon la revendication 1, dans lequel le premier ballonnet dilatable est configuré pour s'engager dans une première lumière corporelle ayant une première section transversale intérieure, et le second ballonnet dilatable est configuré pour s'engager dans une seconde lumière corporelle ayant une seconde section transversale intérieure, la seconde section transversale intérieure étant inférieure à la première section transversale intérieure.

11. Ballonnet d'extraction en deux parties selon la revendication 1, dans lequel le premier ballonnet dilatable a un diamètre de fonctionnement gonflé compris dans une première plage et le second ballonnet dilatable a un diamètre de fonctionnement gonflé compris dans une seconde plage, le premier diamètre de fonctionnement gonflé étant supérieur au second diamètre de fonctionnement gonflé.

12. Ballonnet d'extraction en deux parties selon la revendication 11, dans lequel la première plage de diamètre de fonctionnement gonflé est à peu près 15-20 mm et la seconde plage de diamètre de fonctionnement gonflé est à peu près 8,5-15 mm.

13. Ballonnet d'extraction en deux parties selon la revendication 2, dans lequel le ballonnet d'extraction en deux parties est configuré pour extraire des calculs du canal cholédoque d'un patient, dans lequel la gaine de cathéter a une longueur suffisante pour traverser la papille duodénale majeure et pénétrer dans le canal cholédoque du patient, dans lequel le premier ballonnet dilatable est configuré pour s'appliquer sur la surface intérieure du canal cholédoque du patient quand il est gonflé, et dans lequel le second ballonnet dilatable est configuré pour traverser la papille duodénale majeure quand il est gonflé afin de balayer les calculs dans le duodénum par le sphincter d'Oddi.
